# EUROPEAN PATENT SPECIFICATION

(11) **EP 2 561 794 B1**
(45) Date of publication and mention of the grant of the patent: **21.06.2017**
(21) Application number: 11771880.9
(22) Date of filing: 07.04.2011
(51) Int. Cl.: A61B 1/00, G02B 23/24

(54) **ENDOSCOPE OPERATION SECTION AND ENDOSCOPE**
ENDOSKOPBETRIEBSABSCHNITT UND ENDOSKOP
SECTION DE COMMANDE D'UN ENDOSCOPE ET ENDOSCOPE

(30) Priority: 19.04.2010 JP 2010096201
(43) Date of publication of application: 27.02.2013
(73) Proprietor: Olympus Corporation, Tokyo 192-8507 (JP)
(72) Inventor: NAKAMURA, Naohiro, Hachioji-shi, Tokyo 192-8507 (JP); OGURA, Takeshi, Hachioji-shi, Tokyo 192-8507 (JP)
(74) Representative: Schicker, Silvia
(86) International application number: PCT/JP2011/058802
(87) International publication number: WO 2011/132544

(56) References cited:
- EP-A1- 1 752 082
- JP-A- 9 173 278
- JP-A- 58 102 421
- JP-A- H09 173 278
- JP-A- 2000 267 170
- JP-A- 2002 025 385
- JP-A- 2008 048 803
- JP-U- 63 164 730
- US-A- 4 868 644
- US-A1- 2001 018 551

## Description

### Technical Field

The present invention relates to an endoscope operation section provided at a proximal end of an insertion portion of an endoscope, and an endoscope.

### Background Art

In recent years, endoscopes have widely been used in a medical field and an industrial field. With endoscopes used in the medical field, an elongated insertion portion is inserted into a body cavity, which is a subject, whereby an organ in the body cavity can be observed and as necessary various types of treatments can be performed using a treatment instrument inserted in a treatment instrument insertion channel included in the endoscope.

Also, with endoscopes used in the industrial field, an elongated insertion portion of an endoscope is inserted into an object such as a jet engine or a piping in a plant, whereby observation of, e.g., flaws and corrosion of a site to be inspected in the object or inspection of, e.g., various types of treatments can be performed.

On the proximal end side of an insertion portion of an endoscope, an operation section that is grasped by an operator and includes, e.g., button switches, levers and knobs for performing various types of operation of the endoscope is provided.

More specifically, in the operation section, a grasping region, which is grasped by an operator, is formed on the insertion portion side, and various types of operation members such as bending operation knobs or bending operation levers for performing bending operation of a bending portion provided in the insertion portion, a power supply switch button for the endoscope, a release switch button for providing an instruction to record an image picked up by an image pickup unit, a freeze switch button for providing an instruction to freeze the picked-up image, a zoom switch button for the image pickup unit, switch buttons for air/water sending operation and switch button for suction operation are provided in a region of the operation portion away from the insertion portion relative to the grasping region.

Here, ordinarily, for example, an operator grasps the insertion portion with his right hand in order to send the insertion portion into a subject, and thus, in general, the operation section is grasped by the left hand and the aforementioned various types of operation members provided at the operation section are operated using the respective fingers of the left hand.

However, where switch buttons are arranged side by side at the operation section, the operator who is operating the endoscope while viewing an endoscopic image picked up by the image pickup unit operates the buttons without viewing the operation section, causing the problem of, for example, a switch button that is different from a switch button for desired operation being pushed by a finger of the left hand, that is, a switch being mistakenly operated.

In view of such problem, Japanese Patent Application Laid-Open Publication No. 2002-58629, Japanese Utility Model Application Laid-Open Publication No. 57-84901 and Japanese Patent Application Laid-Open Publication No. 56-72837 each disclose a configuration in which a wall portion is provided between adjacent switch buttons to make an operator recognize the positions of the buttons by mean of the wall portion, preventing erroneous pushing of the buttons.

Some of the aforementioned various types of switch buttons may be provided in a known switch box, which is provided at an end portion of the operation section on the side opposite to the insertion portion in the direction in which the insertion portion is inserted. Also, for operability enhancement, there is a demand for arranging two switch buttons on a same surface of a switch box.

However, if two switch buttons are arranged on a same surface of the switch box as described above and a wall portion is provided between the switch buttons as indicated in Japanese Patent Application Laid-Open Publication No. 2002-58629, Japanese Utility Model Application Laid-Open Publication No. 57-84901 and Japanese Patent Application Laid-Open Publication No. 56-72837 mentioned above, it is necessary to arrange two switch buttons and the wall portion in the same surface of the switch box, causing the problem of an increase in size of the switch box or the wall portion being unable to be provided for space reasons.

Although a configuration in which respective spaces between one of the switch buttons and the wall portion and between the other switch button and the wall portion are narrowed to arrange the two switch buttons on a same surface of a switch box in a space-saving manner may be considered in view of the aforementioned problem, narrowing the spaces between the respective switch buttons and the wall portion is unfavorable because it becomes difficult to clean and disinfect such spaces.

Also, for achieving space saving, the outer diameters of the respective switch buttons may be reduced; however, in this case, there is the problem of a decrease in operability of the switch buttons relative to conventional ones, especially for operators having fat fingers.

Furthermore, when, for example, an operation section is grasped by a left hand in such a manner that a base of a known universal cord extending from a left side of the operation section is held between the thumb and the index finger of the left hand and the chassis of the operation section is grasped by the remaining middle finger, ring finger and little finger, bending operation knobs provided on a right side of the operation section and two switch buttons provided on a front surface of a switch box are operated by the thumb and switch buttons provided on an upper surface and a back surface of the switch box are operated by the index finger and the middle finger.

However, where a wall portion is provided between the two switch buttons provided in the front surface of the switch box, there is the problem that, if the two switch buttons provided in the front surface of the switch box are operated using the thumb moved after operating the bending operation knobs using the thumb, it is easy to push the switch button close to the bending operation knobs while it is very difficult to push the switch button on the side distant from the bending operation knobs because of the existence of the wall portion.

EP 1 752 082 A1 discloses an endoscope comprising an insertion portion. A bending operation knob and image processing unit switches such as a release switch, a freeze switch and an enhance switch are provided on the insertion portion.

JP 2002 025385 A discloses an endoscope comprising an operation mechanism. In the region of a surface of an operation mechanism case, a remote operation button is provided. The remote operation button is fitted in a cylindrical guide cylinder which penetrates through an opening formed in the operation mechanism case. The guide cylinder is provided with a high wall part which is arranged at a side facing an angle lever which serves to prevent the remote operation button from being erroneously pushed when the angle lever is operated in the direction of the remote operation button. The guide cylinder and thus also the high wall part is covered by a button cover.

US 2001/0018551 A1 relates to a manual control part of an endoscope which is provided with a suction button and a freeze switch being arranged in a row on the control part. In the region of its outer circumference, the suction button is encircled with a sleeve.

The present invention has been made in views of the aforementioned circumstances and problems, and an object of the present invention is to provide an endoscope operation section and an endoscope, which include a configuration in which two switch buttons can be arranged side by side in a space-saving manner and misoperation of the switch buttons can be prevented without decreasing the operability of the switch buttons.

### Disclosure of Invention

### Means for Solving the Problem

An endoscope operation section according to an aspect of the present invention is defined in appended claim 1.

### Brief Description of the Drawings

Fig. 1 is a partial perspective view of an endoscope including an endoscope operation section of the present embodiment;
Fig. 2 is a partial perspective view of the endoscope in Fig. 1 taken in the direction of arrow II in Fig. 1;
Fig. 3 is a partial perspective view of the endoscope in Figs. 1 and 2 taken in the direction of arrow III in Figs. 1 and 2;
Fig. 4 is a partial perspective view of the endoscope in Fig. 1 taken in the direction of arrow IV in Fig. 1;
Fig. 5 is a partial perspective view of the endoscope in Fig. 1 taken in the direction of arrow V in Fig. 1;
Fig. 6 is a partial perspective view of an enlargement of an area in the vicinity of a first switch button and a second switch button in the endoscope operation section in Fig. 1;
Fig. 7 is a partial perspective view illustrating a state in which the endoscope operation section in Fig. 1 is grasped by the left hand of an operator;
Fig. 8 is a partial cross-sectional view of an endoscope operation section taken along line VIII-VIII in Fig. 6, centering on the second switch button; and
Fig. 9 is an enlarged partial perspective view of an operation section according to a variation in which a projection portion provided on an outer circumference of a second switch button extends by a set length toward an insertion portion.

### Best Mode for Carrying Out the Invention

An embodiment of the present invention will be described below with reference to the drawings. It should be noted that the drawings are schematic ones and, e.g., the relationship between a thickness and a width of each member and the ratios in thickness between the respective members are different from actual ones, and it should be understood that the drawings include parts that are different in dimensional relationship and/or ratio from one another.

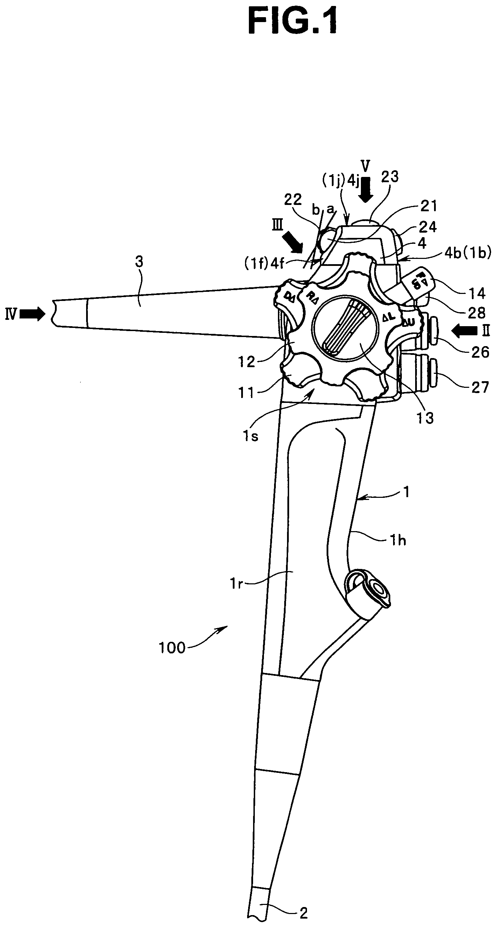

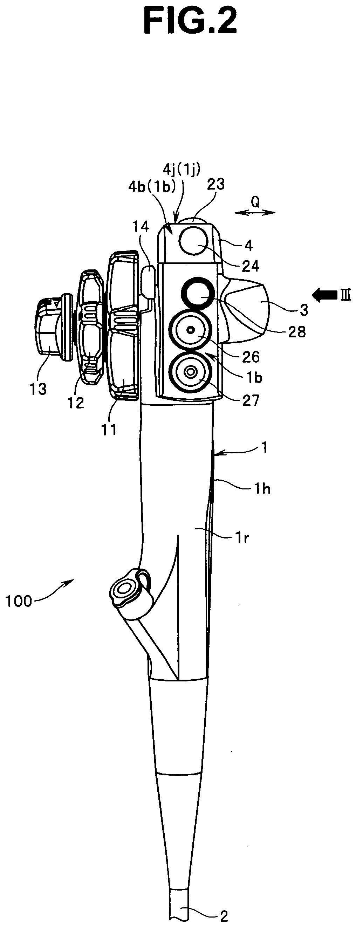

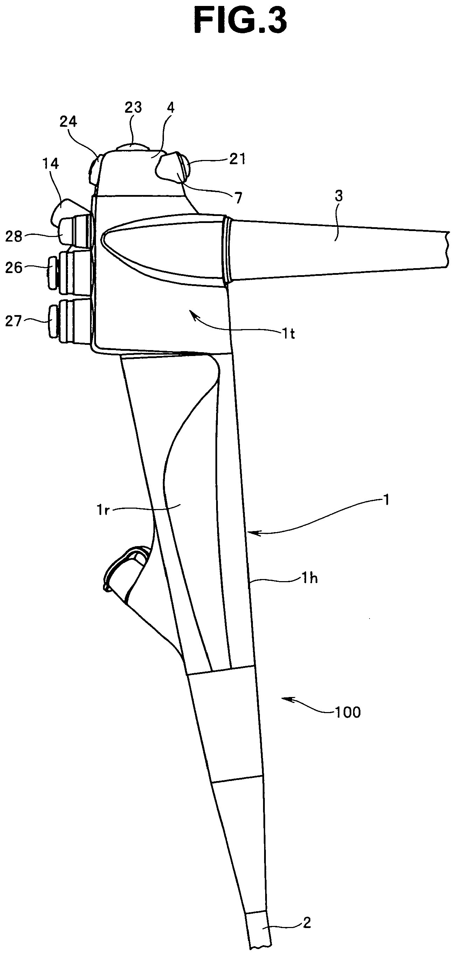

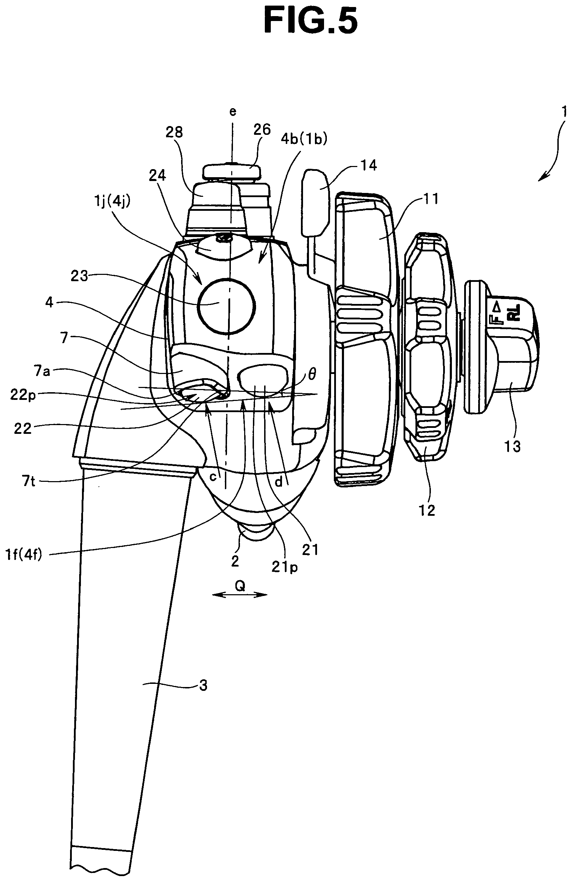

Fig. 1 is a partial perspective view of an endoscope including an endoscope operation section according to the present embodiment; Fig. 2 is a partial perspective view of the endoscope in Fig. 1 taken in the direction of arrow II in Fig. 1; Fig. 3 is a partial perspective view of the endoscope in Figs. 1 and 2 taken in the direction of arrow III in Figs. 1 and 2; Fig. 4 is a partial perspective view of the endoscope in Fig. 1 taken in the direction of arrow IV in Fig. 1; and Fig. 5 is a partial perspective view of the endoscope in Fig. 1 taken in the direction of arrow V in Fig. 1.

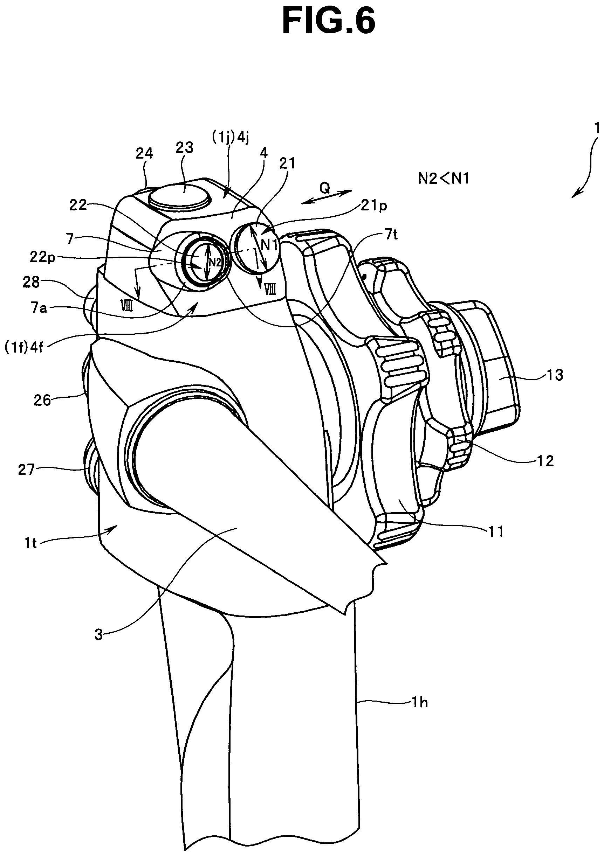

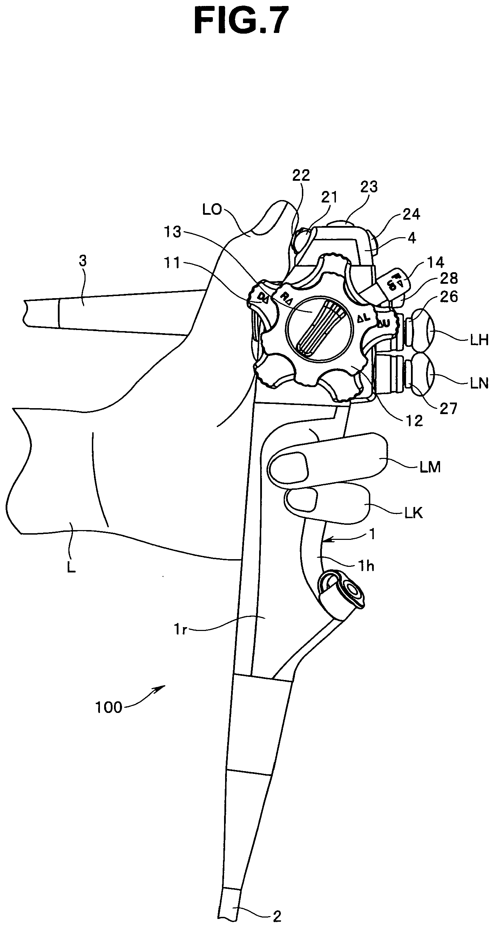

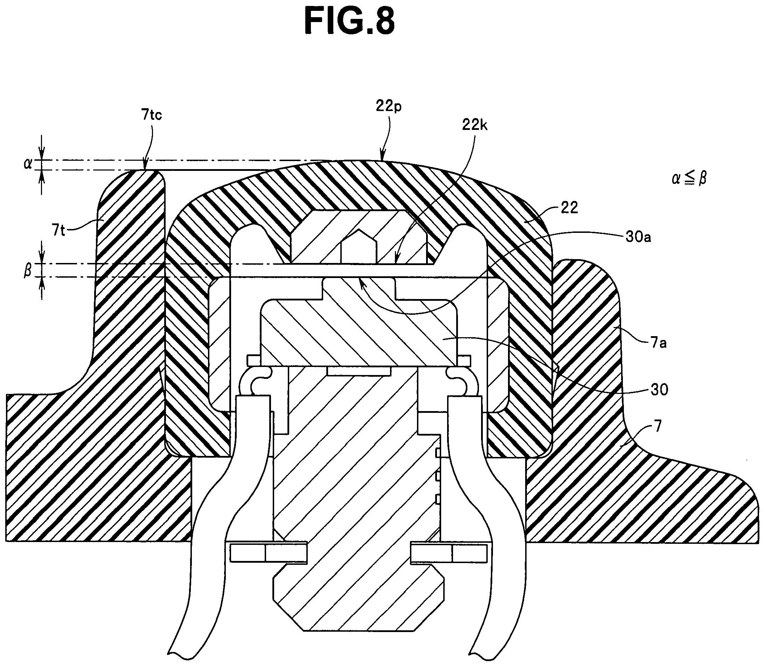

Also, Fig. 6 is a partial perspective view of an enlargement of an area in the vicinity of a first switch button and a second switch button in the endoscope operation section in Fig. 1; Fig. 7 is a partial perspective view of a state in which the endoscope operation section in Fig. 1 is grasped by the left hand of an operator; and Fig. 8 is a partial cross-sectional view of the endoscope operation section taken along line VIII-VIII in Fig. 6, centering on the second switch button.

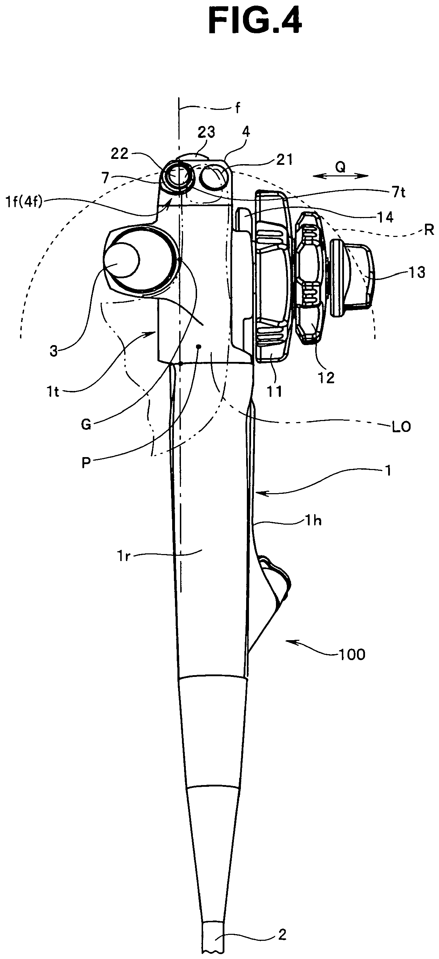

As illustrated in Fig. 1, a main portion of an endoscope 100 includes an insertion portion 2 to be inserted into a subject, an endoscope operation section (hereinafter simply referred to as the operation section) 1 provided on the proximal end side of the insertion portion 2, a universal cord 3 extending from a third surface 1t (see Figs. 3 and 4) adjacent to a first surface 1f of the operation section 1, which will be described later, and a non-illustrated connector provided at an extension end of the universal cord 3.

In an operation section body 1h of the operation section 1, as illustrated in Fig. 7, a grasping region 1r, which is grasped by the palm, the ring finger LM and the little finger LK of the left hand L of an operator, is formed on the insertion portion 2 side, and various types of operation switches are provided in a region of the operation section body 1h on the side away from the insertion portion 2 relative to the grasping region 1r (hereinafter referred to as the upper side).

More specifically, as illustrated in Figs. 1 to 7, a switch box 4 is provided at an end portion of the operation section body 1h that is away from the insertion portion 2 (hereinafter referred to as the upper portion).

As illustrated in Figs. 1 and 4 to 7, a first switch button 21 for performing first operation of the endoscope 100 and a second switch button 22 for performing second operation of the endoscope 100 are provided on a first surface 4f of the switch box 4, which is included in the first surface 1f of the operation section body 1h.

The first switch button 21 may be a frequently-used switch button, for example, a release switch button for providing an instruction to record an image picked up by a non-illustrated image pickup unit provided in a distal end of the insertion portion 2.

Also, the second switch button 22 may be a less frequently-used switch button, for example, a zoom switch button for the aforementioned image pickup unit. In other words, in this case, the first operation is operation for an image recording instruction and the second instruction is zoom operation. Also, configurations and arrangement of the first switch button 21 and the second switch button 22 will be described later.

Also, as illustrated in Figs. 1 to 7, for example, a power switch button 23 for turning on/off a power supply of the endoscope 100 is provided on an upper surface 4j of the switch box 4 included in an upper surface 1j of the operation section body 1h.

Furthermore, as illustrated in Figs. 1 to 7, for example, an iris switch button 24 for changing an exposure measuring method is provided on a back surface 4b of the switch box 4 included in a back surface 1b of the operation section body 1h.

Also, as illustrated in Fig. 5, the power supply switch button 23 and the iris switch button 24 may be provided offset from a center e of the switch box in a direction away from bending operation knobs 11 and 12, which will be described later, in a direction Q in which the first switch button 21 and the second switch button 22 are arranged side by side, in the upper surface 4j and the back surface 4b of the switch box 4, respectively.

A reason for this is: as illustrated in Fig. 7, when the operation section body 1h is grasped by a left hand L in such a manner that a base of the universal cord 3 extending from the third surface 1t of the operation section body 1h is held between the thumb LO and the index finger LH of the left hand L, the power supply switch button 23 and the iris switch button 24 are operated by the index finger LH. Accordingly, in this way of grasping, if the power supply switch button 23 and the iris switch button 24 are provided offset in the direction away from the bending operation knobs 11 and 12 in the direction Q, distances from the respective switches 23 and 24 to the index finger LH become short, facilitating operation by the index finger LH.

As illustrated in Figs. 1 and 2 and Figs. 4 to 7, the bending operation knobs 11 and 12, which are angle operation members for bending a non-illustrated bending portion provided in the insertion portion 2, are provided at a site of a second surface 1s that is adjacent to the first surface 1f and faces the third surface 1t, the site being on the upper side relative to the grasping region 1r of the operation section body 1h and on the insertion portion 2 side (hereinafter referred to as the lower side) relative to the switch box 4.

The angle operation members are not limited to knobs and may be other operation members such as levers and/or switch buttons. Also, where the first surface 4f of the switch box 4 is very large, the angle operation members may be provided in the first surface 4f.

The bending operation knob 11 is a knob that is operated to rotate in order to vertically bend the aforementioned bending portion, and a rotational position thereof is fixed by means of operation of a rotational position fixing lever 14.

Also, the bending operation knob 12 is a knob that is operated to rotate in order to horizontally bend the aforementioned bending portion, and a rotational position thereof is fixed by means of operation of a rotational position fixing dial 13.

As illustrated in Fig. 7, when the operation section body 1h is grasped by the left hand L in such a manner that the base of the universal cord 3 is held between the thumb LO and the index finger LH of the left hand L, the bending operation knobs 11 and 12 are operated by the thumb LO.

As illustrated in Figs. 1 to 3 and Figs. 5 to 7, for example, a freeze switch button 28 for providing an instruction to freeze an image picked up by the image pickup unit, a known switch button 26 for air/water sending operation and a switch button 27 for suction operation are provided at a site of the back surface 1b of the operation section body 1h, the site being on the upper side relative to the grasping region 1r and on the lower side relative to the switch box 4.

As illustrated in Fig. 7, when the operation section body 1h is held by the left hand L in such a manner that the base of the universal cord 3 is held between the thumb LO and the index finger LH of the left hand L, the freeze switch button 28, the switch button 26 for air/water sending operation and the switch button 27 for suction operation are operated by the index finger LH and the middle finger LN.

Next, configurations and arrangement of the first switch button 21 and the second switch button 22 will be described.

As illustrated in Figs. 4 to 6, the first switch button 21 and the second switch button 22 are arranged side by side along a direction Q in the first surface 4f of the switch box 4, the second switch button 22 is positioned on the side distant from the bending operation knobs 11 and 12 in the direction Q relative to the first switch button 21.

Also, the first switch button 21 and the second switch button 22 are arranged at positions in the first surface 4f where the first switch button 21 and the second switch button 22 are operated by the thumb LO when the operation section body 1h is grasped by the left hand L in such a manner that the base of the universal cord 3 is held between the thumb LO and the index finger LH of the left hand L as illustrated in Fig. 7.

Furthermore, the first switch button 21 and the second switch button 22 are arranged in the first surface 4f so as to be positioned on an outer circumference R of a circle with the base P of the thumb LO as a center as illustrated in Fig. 4 when the operation section body 1h is grasped by the left hand L in such a manner that the base of the universal cord 3 is held by the thumb LO and the index finger LH of the left hand L as illustrated in Fig. 7.

Consequently, mere movement of the thumb LO along the direction Q enables pushing operation of the first switch button 21 and the second switch button 22 to be performed by the thumb LO, facilitating the pushing operation of the first switch button 21 and the second switch button 22.

Also, as illustrated in Fig. 4, the second switch button 22 is arranged in the first surface 4f so as to be positioned on a line f extending from a point G where the thumb LO abuts to the universal cord 3 in a direction in which the operation section body 1h extends when the operation section body 1h is grasped by the left hand L in such a manner that the base of the universal cord 3 is grasped between the thumb LO and the index finger LH of the left hand L as illustrated in Fig. 7. Also, the second switch button 22 may be arranged on the side away from the bending operation knobs 11 and 12 in the direction Q relative to the line f.

As illustrated in Figs. 4 to 6, a part on an outer circumferential side of the first switch button 21 is covered by a sheathing member providing the first surface 4f of the switch box 4, and a pushing surface 21p is exposed.

As illustrated in Figs. 4 to 6, a part on an outer circumferential side of the second switch button 22 is covered by the sheathing member providing the first surface 4f of the switch box 4. More specifically, the part on the outer circumferential side of the second switch button 22 is covered by the second switch button 22 being fitted in a fitting portion 7 formed in the sheathing member, and the pushing surface 22p is exposed.

Like the first switch button 21, the part on the outer circumferential side of the second switch button 22 may be covered by the sheathing member providing in the first surface 4f of the switch box 4 alone.

Also, a projection portion 7t is formed at a site of the fitting portion 7, the site facing the first switch button 21 in the one direction Q, on the outer circumference of the second switch button 22, the projection portion 7t projecting toward the side away from the first surface 4f relative to another site 7a of the fitting portion 7.

The projection portion 7t is a member that prevents the first switch button 21 or the second switch button 22 from being pushed in error for the second switch button 22 or the first switch button 21 by the thumb LO and makes the operator who cannot see the operation section body 1h during operation recognize a position of the thumb LO when the operation section body 1h is held by the left hand L in such a manner that the base of the universal cord 3 is held between the thumb LO and the index finger LH of the left hand L as illustrated in Fig. 7.

The projection portion 7t is provided only at the site of the fitting portion 7 that faces the first switch button 21 in the direction Q, not on the entire outer circumference of the second switch button 22 because if the projection portion 7t is provided on the entire circumference, the projection portion 7t provided on the entire circumference becomes an obstacle for operators with a large thumb LO, making it difficult to perform pushing operation of the second switch button 22.

Also, a height of a projection of the projection portion 7t is set as indicated in Fig. 8. More specifically, where a gap between an upper surface 7tc of the projection portion 7t and an apex of the pushing surface 22p of the second switch button 22 is set as α and a gap between a switch surface 30a of an electronic switch 30 provided in the second switch button 22 and a switch-on/off surface 22k of the second switch button 22 is set as β, it is only necessary that α ≤ β. In other words, it is only necessary that the upper surface 7tc be higher than a value resulting from β being subtracted from the apex of the pushing surface 22p.

A reason for this: where α ≤ β, even if the thumb LO mistakenly touches the apex of the pushing surface 22p when the thumb LO slides in the direction Q to move from the first switch button 21 to the projection portion 7t, the switch surface 30a is not turned on by the on/off surface 22k even when the pushing surface 22p is pushed up to the upper surface 7tc, and use of this enables effective prevention of erroneous pushing of the second switch button 22.

The above applies also to the first switch button 21. In other words, although there is no projection portion on an outer circumference of the first switch button 21, where a gap between an apex of the pushing surface 21p of the first switch button 21 and the upper surface 7tc of the projection portion 7t provided on the outer circumference of the second switch button 22 is set as α', and a gap between a switch surface of an electronic switch provided in the first switch button 21 and a switch-on/off surface of the first switch button 21 is set as β', if α' ≤ β', erroneous pushing of the first switch button 21 can effectively be prevented when the thumb LO slides to move from the second switch button 22 to the projection portion 7t in the direction Q.

Also, as illustrated in Fig. 6, an outer diameter N2 of the second switch button 22 is formed so as to be smaller than an outer diameter N1 of the first switch button 21 (N1 > N2).

More specifically, the outer diameter N1 of the first switch button 21 is formed so as to have a size equivalent to those of conventional release switch buttons, and the outer diameter N2 of the second switch button 22 is formed so as to have a size somewhat smaller than those of the conventional release switch buttons.

A reason for this is: since a frequently-used button function, for example, a release function, is assigned to the first switch button 21 as described above, if the outer diameter N1 of the first switch button 21 is small, the usability deteriorates compared to conventional ones; however, since a less frequently-used button function, for example, a zoom function, is assigned to the second switch button 22, there is no large impact on the usability even if the outer diameter N2 of the second switch button 22 is small.

Another reason is: since the area of the first surface 4f of the switch box 4 is limited, in order to provide two switch buttons 21 and 22 in a space-saving manner, it is inevitable to reduce the size of the less frequently-used button.

Accordingly, if it can be ensured that the first surface 4f has a large area, it is not necessary that the outer diameter N2 of the second switch button 22 be smaller than the outer diameter N1 of the first switch button 21.

Also, as indicated by arrows c and d in Fig. 5, the pushing surfaces 21p and 22p of the first switch button 21 and the second switch button 22 are positioned at a tilt of a set angle θ, for example, 7° toward the bending operation knobs 11 and 12 relative to the first surface 4f of the switch box 4.

A reason for this is: when the operation section body 1h is grasped by the left hand L in such a manner that the base of the universal cord 3 is held between the thumb LO and the index finger LH of the left hand L as illustrated in Fig. 7, if the thumb LO is moved in the direction Q to perform pushing operation of the first switch button 21 and the second switch button 22 after the bending operation knobs 11 and 12 are operated by the thumb LO, as a result of the pushing surfaces 21p and 22p being positioned at a tilt of the set angle θ toward the bending operation knobs 11 and 12 relative to the first surface 4f, a distance of movement of the thumb LO from the bending operation knobs 11 and 12 to the first switch button 21 and the second switch button 22 is reduced, facilitating the pushing operation of the first switch button 21 and the second switch button 22.

Another reason is: when the operation section body 1h is grasped by the left hand L in such a manner that the base of the universal cord 3 is held between the thumb LO and the index finger LH of the left hand L as illustrated in Fig. 7, the thumb LO touches the universal cord 3 at the point G as illustrated in Fig. 4, and thus, tilting of, in particular, the second switch button 22 toward the bending operation knobs 11 and 12 facilitates the pushing operation of the second switch button 22.

Also, as illustrated in Fig. 1, the first switch button 21 and the second switch button 22 are positioned at a tilt of a set angle from the first surface 4f of the switch box 4 in the direction in which the operation section body 1h extends, and as indicated by lines a and b, the second switch button 22 is positioned in such a manner that the second switch button 22 is more tilted in the direction in which the operation section body 1h extends than the first switch button 21. In other words, the angles of the tilting of the first switch button 21 and the second switch button 22 in the direction in which the operation section body 1h extends are different from each other.

Furthermore, the second switch button 22 projects from the first surface 4f of the switch box 4 higher than the first switch button 21. In other words, the pushing surface 22p is positioned to be higher than the pushing surface 21p.

A reason of this is: when the operation section body 1h is grasped by the left hand L in such a manner that the base of the universal cord 3 is held between the thumb LO and the index finger LH of the left hand L as illustrated in Fig. 7, if the second switch button 22, which is positioned on the line f or on the side away from the bending operation knobs 11 and 12 in the direction Q relative to the line f as illustrated in Fig. 4, is operated, the thumb LO touches the universal cord 3 at the point G; however, if the second switch button 22 is formed to have a same height as that of the first switch button 21 or is tilted at a same angle of that of the first switch button 21 in the direction in which the operation section body 1h extends, a distance from the thumb LO to the second switch button 22 is longer than a distance from the thumb LO to the first switch button 21, making it difficult to push the second switch button 22.

Accordingly, making the second switch button 22 higher than the first switch button 21 or the second switch button 22 being tilted more than the first switch button 21 in the direction in which the operation section body 1h extends provides the effect of making it easy for the thumb LO to reach the second switch button 22.

Also, even where the first switch button 21 and the second switch button 22 are positioned on the outer circumference R with the root P of the thumb LO as a center in the first surface 4f of the switch box 4 as illustrated in Fig. 4 mentioned above, if the second switch button 22 is positioned to be lower than the first switch button 21 as opposed to the above description, the first switch button 21 may mistakenly be pushed when the thumb LO is moved in the direction Q to operate the second switch button 22 after operating the bending operation knobs 11 and 12.

Accordingly, it is necessary to ensure that the second switch button 22 is higher than the first than switch button 21; however, since provision of the aforementioned projection portion 7t on the outer circumference of the second switch button 22 enables prevention of erroneous pushing as described above, the pushing surface 22p of the second switch button 22 and the pushing surface 21p of the first switch button 21 may have a same height.

As described above, in the present embodiment, a case where the projection portion 7t is formed at the site of the fitting portion 7, the site facing the first switch button 21 in the direction Q, on the outer circumference of the second switch button 22, the projection portion 7t projecting toward the side away from the first surface 4f relative to the other site 7a of the fitting portion 7, has been indicated.

In this case, an operator can easily recognize positions of the first switch button 21 and the second switch button 22 by means of the projection portion 7t without seeing the operation section body 1h even after operating the bending operation knobs 11 and 12 in a configuration in which the bending operation knobs 11 and 12, the first switch button 21 and the second switch button 22 are operated using a thumb LO, enabling prevention erroneous pushing of the buttons.

Furthermore, although in a conventional configuration, a wall for erroneous pushing prevention is provided between switch buttons and it is thus necessary to ensure a space between one of the switch buttons and the wall and a space between the other switch button and the wall, making it impossible to provide two switch buttons in a space-saving manner, in the present configuration, a projection portion, which serves as a wall portion, is the projection portion 7t of the fitting portion 7 covering the outer circumferential side of the second switch button 22, and it is thus only necessary to ensure a space for the first switch button 21 and the second switch button 22 and the fitting portion 7, enabling two switch buttons 21 and 22 to be provided side by side along the direction Q in the first surface 4f of the switch box 4.

Furthermore, in the present embodiment, a case where a frequently-used function is assigned to the first switch button 21, which is close to the bending operation knobs 11 and 12, and a less frequently-used function is assigned to the second switch button 22 positioned on the side distant from the bending operation knobs 11 and 12 in the direction Q has been indicated.

Also, a case where the outer diameter N1 of the first switch button 21 has a size that is the same as outer diameters of conventional release switch buttons and the outer diameter N2 of the second switch button 22 is formed so as to have a size smaller than the outer diameter N1 has been indicated.

In these cases, the operability of the first switch button 21 to which a frequently-used release button function is assigned can be ensured to be equivalent to that of the conventional ones. Meanwhile, the projection portion 7t is formed on the less frequently-used second switch button 22 and the outer diameter N2 of the second switch button 22 is formed to be smaller, making it difficult to push the button 22 and the operability of the second switch button 22 is decreased compared to that of the conventional ones; however, a less frequently-used function is assigned to the second switch button 22 and the second switch button 22 is thus less frequently operated, enabling the decrease of the operability to be suppressed to the minimum.

Furthermore, the projection portion 7t is formed only at a site of the fitting portion 7 that faces the first switch button 21, preventing making it difficult to push the second switch button 22, compared to a case where the projection portion 7t is formed on the entire circumference.

According to the above, an operation section 1 including a configuration in which two switch buttons 21 and 22 are arranged side by side in a space-saving manner, the configuration enabling prevention of misoperation of the switch buttons 21 and 22 without a decrease in operability of the switch buttons 21 and 22 can be provided.

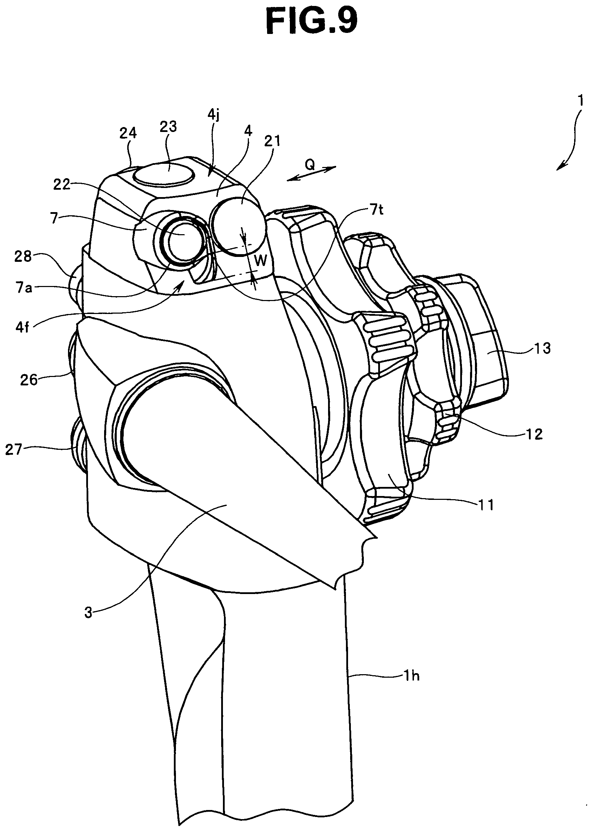

A variation will be described below with reference to Fig. 9. Fig. 9 is an enlarged partial perspective view of an operation section according to a variation in which a projection portion provided on an outer circumference of a second switch button extends by a set length toward the insertion portion.

As illustrated in Fig. 9, a projection portion 7t projecting toward the side away from a first surface 4f at a site of a fitting portion 7, the site facing a first switch button 21 in one direction Q, on an outer circumference of a second switch button 22 relative to another site 7a of the fitting portion 7 may be formed so as to extend in the first surface 4 of a switch box 4 by a predetermined length w toward the insertion portion 2.

Consequently, when an operation section body 4h is grasped by a left hand L in such a manner that a base of a universal cord 3 is held between the thumb LO and the index finger LH of the left hand L as illustrated in Fig. 7, even an operator with a short thumb LO can make the thumb LO touch the projection portion 7t and thus can recognize the projection portion 7t, making it easy to know which of the first switch button 21 and the second switch button 22 the finger should reach for, enabling prevention of erroneous pushing of the switch buttons 21 and 22.

Furthermore, in the present embodiment described above, the projection portion is formed only at a part of the outer circumference of the second switch button 22, and thus, if a thumb LO is made to touch the projection portion 7t for a long period of time or the thumb LO is made to touch the projection portion 7t many times, the operator may feel pain; however, making the surface area of the projection portion 7t larger than that of the present embodiment as illustrated in Fig. 9 to disperse the pressure on the thumb LO, enabling reduction of the pain of the thumb LO touching the projection portion 7t.

The rest of the effects is similar to those of the present embodiment. Furthermore, in the present embodiment described above, a case where the first switch button 21 is used as a release switch button and the second switch button 22 is used as a zoom switch button, and the switch button 23 is used as a power supply switch button and the switch button 24 is used as an iris switch button, and the switch 28 is used as a freeze switch has been indicated; however, the present invention is not limited to this case, and it should be understood that the functions of the switches are assigned in a desired manner so as to be convenient for users. However, even in this case, it is preferable that a less frequently-used button be assigned to the second switch button 22.

Furthermore, a case where in the angle operation members are provided in the second surface 1s of the operation section body and the first switch button 21 and the second switch button 22 are provided in the first surface 1f of the operation section body has been indicated; however, these members and buttons may be provided on a same surface. However, in this case, it is necessary that the angle operation members, the first switch button 21 and the second switch button 22 be arranged side by side and it is also necessary that the second switch button 22 be provided on the side distant from the angle operation member.

The present application is filed claiming the priority of Japanese Patent Application No. 2010-096201 filed in Japan on April 19, 2010.

## Claims

1. An endoscope operation section (1) comprising:
an angle operation member (11, 12) for bending a bending portion provided in an insertion portion (2) of an endoscope (100), the angle operation member (11, 12) being provided in an operation section body (1h) of the endoscope operation section (1) provided at a proximal end of the insertion portion (2);
a first switch button (21) for performing first operation of the endoscope (100), the first switch button (21) being provided in a first surface (1f) of the operation section body (1h); and
a second switch button (22) for performing second operation of the endoscope (100), the second switch button (22) being provided side by side with the first switch button (21) in the first surface (1f) and positioned on a side distant from the angle operation member (11, 12) relative to the first switch button (21),
wherein a universal cord (3) extends from a third surface (1t) that is adjacent to the first surface (1f) of the operation section body (1h) and faces a second surface(1s) adjacent to the first surface (1f), wherein the first switch button (21) and the second switch button (22) are formed at positions of the first surface (1f) where the first switch button (21) and the second switch button (22) are suitable of being operated by a thumb when the operation section body (1h) is grasped by one hand in such a manner that a base of the universal cord (3) is held between the thumb and an index finger, and wherein the angle operation member is provided in the second surface,
**characterized in that** an outer circumferential side of the second switch button (22) is covered by a sheathing member of the operation section body (1h), and a projection portion (7t) of the sheathing member is formed at a site of the sheathing member facing the first switch button (21), on an outer circumference of the second switch button (22), the projection portion (7t) projecting toward a side away from the first surface (1f) relative to another site of the sheathing member on the outer circumference of the second switch button (22).

2. The endoscope operation section according to claim 1,
wherein the second switch button (22) is fitted in a fitting portion (7) formed in the sheathing member; and
wherein the projection portion (7t) is formed at a site of the fitting portion (7), the site facing the first switch button (21).

3. The endoscope operation section according to any one of claims 1 to 2, wherein the projection portion (7t) is formed so as to extend in the first surface (1f) by a predetermined length toward the insertion portion (2) from the outer circumference of the second switch button (22).

4. An endoscope (100) comprising the endoscope operation section (1) according to any one of claims 1 to 3.

## Patentansprüche

1. Endoskopbetätigungsabschnitt (1), der umfasst:
ein Winkelbetätigungselement (11, 12), um einen in einem Einführabschnitt (2) eines Endoskops (100) vorgesehenen Biegeabschnitt zu biegen, wobei das Winkelbetätigungselement (11, 12) in einem an einem proximalen Ende des Einführabschnitts (2) vorgesehenen Betätigungsabschnittskörper (1h) des Endoskopbetätigungsabschnitts (1) vorgesehen ist;
einen ersten Schaltknopf (21), um eine erste Betätigung des Endoskops (100) auszuführen, wobei der erste Schaltknopf (21) in einer ersten Oberfläche (1f) des Betätigungsabschnittskörpers (1h) vorgesehen ist; und
einen zweiten Schaltknopf (22), um eine zweite Betätigung des Endoskops (100) auszuführen, wobei der zweite Schaltknopf (22) in der ersten Oberfläche (1f) neben dem ersten Schaltknopf (21) vorgesehen und relativ zu dem ersten Schaltknopf (21) auf einer von dem Winkelbetätigungselement (11, 12) entfernten Seite angeordnet ist,
wobei sich ein Universalkabel (3) von einer dritten Oberfläche (1t) erstreckt, die benachbart zu der ersten Oberfläche (1f) des Betätigungsabschnittkörpers (1h) und einer der zu der ersten Oberfläche (1f) benachbarten zweiten Oberfläche (1s) zugewandt ist, wobei der erste Schaltknopf (21) und der zweite Schaltknopf (22) an Positionen der ersten Oberfläche (1f) gebildet sind, wo der erste Schaltknopf (21) und der zweite Schaltknopf (22) geeignet sind, durch einen Daumen bedient zu werden, wenn der Betätigungsabschnittskörper (1h) durch eine Hand so gegriffen wird, dass eine Basis des Universalkabels (3) zwischen dem Daumen und einem Zeigefinger gehalten wird, und wobei das Winkelbetätigungselement in der zweiten Oberfläche vorgesehen ist,
**dadurch gekennzeichnet, dass** eine Außenumfangsseite des zweiten Schaltknopfs (22) durch ein Verkleidungselement des Betätigungsabschnittskörpers (1h) bedeckt ist, und ein Vorsprungsabschnitt (7t) des Verkleidungselements an einem Außenumfang des zweiten Schaltknopfs (22) an einer dem ersten Schaltknopf (21) zugewandten0 Stelle des Verkleidungselements gebildet ist, wobei der Vorsprungsabschnitt (7t) an dem Außenumfang des zweiten Schaltknopfs (22) relativ zu einer anderen Stelle des Verkleidungselements in Richtung zu einer von der ersten Oberfläche (1f) abgewandten Seite hervorsteht.

2. Endoskopbetätigungsabschnitt nach Anspruch 1,
wobei der zweite Schaltknopf (22) in einen im Verkleidungselement gebildeten Passabschnitt (7) eingefügt ist; und
wobei der Vorsprungsabschnitt (7) an einer Stelle des Passabschnitts (7) gebildet ist, wobei die Stelle dem ersten Schaltknopf (21) zugewandt ist.

3. Endoskopbetätigungsabschnitt nach einem der Ansprüche 1 oder 2,
wobei der Vorsprungsabschnitt (7) so geformt ist, dass er sich in der ersten Oberfläche (1f) um eine vorbestimmte Länge von dem Außenumfang des zweiten Schaltknopfs (22) in Richtung des Einführabschnitts (2) erstreckt.

4. Endoskop (100), das einen Endoskopbetätigungsabschnitt (1) nach einem der Ansprüche 1 bis 3 umfasst.

## Revendications

1. Section (1) de commande d'endoscope comprenant :
un élément (11, 12) de commande d'angle destiné à courber une partie de courbure prévu dans une partie d'insertion (2) d'un endoscope (100), l'élément (11, 12) de commande d'angle étant prévu dans un corps (1h) de section de commande de la section (1) de commande d'endoscope prévu au niveau d'une extrémité proximale de la partie d'insertion (2) ;
un premier bouton commutateur (21) destiné à exécuter une première commande de l'endoscope (100), le premier bouton commutateur (21) étant prévu dans une première surface (1f) du corps (1h) de section de commande ; et
un deuxième bouton commutateur (22) destiné à exécuter une deuxième commande de l'endoscope (100), le deuxième bouton commutateur (22) étant prévu côte à côte avec le premier bouton commutateur (21) dans la première surface (1f) et positionné sur un côté distant de l'élément (11, 12) de commande d'angle par rapport au premier bouton commutateur (21),
dans laquelle un cordon universel (3) s'étend depuis une troisième surface (1t) qui est adjacente à la première surface (1f) du corps (1h) de section de commande et fait face à une deuxième surface (1s) adjacente à la première surface (1f),
dans laquelle le premier bouton commutateur (21) et le deuxième bouton commutateur (22) sont formés à des positions de la première surface (1f) au niveau desquelles le premier bouton commutateur (21) et le deuxième bouton commutateur (22) sont appropriés pour être actionnés par un pouce lorsque le corps (1h) de section de commande est saisi par une main d'une manière telle qu'une base du cordon universel (3) est maintenue entre le pouce et un index, et dans laquelle l'élément de commande d'angle est prévu dans la deuxième surface,
**caractérisé en ce qu'**un côté circonférentiel externe du deuxième bouton commutateur (22) est recouvert par un élément de gainage du corps (1h) de section de commande, et une partie faisant saillie (7t) de l'élément de gainage est formée au niveau d'un emplacement de l'élément de gainage faisant face au premier bouton commutateur (21), sur une circonférence externe du deuxième bouton commutateur (22), la partie faisant saillie (7t) faisant saillie vers un côté éloigné de la première surface (1f) par rapport à un autre emplacement de l'élément de gainage sur la circonférence externe du deuxième bouton commutateur (22).

2. Section de commande d'endoscope selon la revendication 1,
dans laquelle le deuxième bouton commutateur (22) est ajusté dans une partie d'ajustement (7) formée dans l'élément de gainage ; et
dans laquelle la partie faisant saillie (7t) est formée au niveau d'un emplacement de la partie d'ajustement (7), l'emplacement faisant face au premier bouton commutateur (21).

3. Section de commande d'endoscope selon l'une quelconque des revendications 1 à 2,
dans laquelle la partie faisant saillie (7t) est formée de façon à s'étendre dans la première surface (1f) d'une longueur prédéterminée vers la partie d'insertion (2) depuis la circonférence externe du deuxième bouton commutateur (22).

4. Endoscope (100) comprenant la section (1) de commande d'endoscope selon l'une quelconque des revendications 1 à 3.
